Europäisches Patentamt

European Patent Office

Office européen des brevets

(19)

(11) Publication number: **0 269 072 B1**

# EUROPEAN PATENT SPECIFICATION

(12)

(45) Date of publication of patent specification: **23.09.92**  (51) Int. Cl.[5]: **C12N 15/00**, //C12P21/02

(21) Application number: **87117325.8**

(22) Date of filing: **24.11.87**

(54) Cloning of DNA encoding human gip precursor and expression of the precursor.

(30) Priority: **27.11.86 JP 282812/86**

(43) Date of publication of application:
**01.06.88 Bulletin 88/22**

(45) Publication of the grant of the patent:
**23.09.92 Bulletin 92/39**

(84) Designated Contracting States:
**CH DE FR GB IT LI**

(56) References cited:

**FEBS, vol. 172, no. 2, July 1984, pages
142-148, Elsevier Science Publishers B.V.
Amsterdam, NL; A.J. MOODY et al.: "The
isolation and sequencing of human gastric
inhibitory peptide (GIP)"**

**ANGEWANDTE CHEMIE INTERNATIONAL EDI-
TION, vol. 22, 1983, pages 842-858, Verlag
Chemie GmbH, Weinheim, DE; F. WENGEN-
MAYER: " Synthesis of peptide hormones
using recombinant DNA techniques"**

**PEPTIDES, vol. 7, Suppl. 1, 1986, pages 27-36,
Ankho International Inc., US; G.I. BELL: "The
glucagon superfamily: Precursor structure
and gene organization"**

(73) Proprietor: **SANWA KAGAKU KENKYUSHO
CO., LTD.
No. 35, Higashi-sotobori-cho
Higashi-ku Nagoya-shi Aichi-ken(JP)**

(72) Inventor: **Takeda, Jun
18, Shogoin-Sannno-cho
Sakyo-ku Kyoto(JP)**
Inventor: **Imura, Hiroo
59-2, Ichijoji Kita-ohmaru-cho
Sakyo-ku Kyoto(JP)**
Inventor: **Seino, Yutaka
4-8-20, Tomatsu-cho Amagasaki-shi
Hyogo-ken(JP)**
Inventor: **Tanaka, Kenichi c/o Sanwa Kagaku
Kenkyusho Co., Lt
35, Higashi-sotobori-cho Higashi-ku
Nagoya-shi Aichi-ken(JP)**
Inventor: **Takahashi, Haruo c/o Sanwa Kagaku
Kenkyusho Co.,
35, Higashi-sotobori-cho Higashi-ku
Nagoya-shi Aichi-ken(JP)**
Inventor: **Mitani, Takahiko c/o Sanwa Kagaku
Kenkyusho Co.,
35, Higashi-sotobori-cho Higashi-ku
Nagoya-shi Aichi-ken(JP)**

PROCEEDINGS OF THE NATIONAL ACADEMY OF SCIENCES OF USA, vol. 84, October 1987, pages 7005-7008, Washington DC, US; J. TAKEDA et al.: "Sequence of an intestinal cDNA encoding human gastric inhibitory polypeptide precursor"

Inventor: **Kurono, Masayasu c/o Sanwa Kagaku Kenyusho Co.,**
**35, Higashi-sotobori-cho Higashi-ku**
**Nagoya-shi Aichi-ken(JP)**
Inventor: **Sawai, Kiichi c/o Sanwa Kagaku Kenkyusho Co., Ltd.**
**35, Higashi-sotobori-cho Higashi-ku**
**Nagoya-shi Aichi-ken(JP)**

(74) Representative: **Wächtershäuser, Günter, Dr.**
**Tal 29**
**W-8000 München 2(DE)**

**Description**

The present invention relates to a gastric inhibitory polypeptide (referred to as --GIP--), and more particularly to a cloned DNA of human GIP precursor, its fragment, a process for the preparation of the cloned DNA or its fragment, as well as a plasmid, in which the DNA or its fragment is integrated therein.

The GIP is one of the peptide hormones, presents in stomach, intestines, pancreas and other organs, and firstly isolated by Brown, J. C. et al from porcine duodenum ["J. Physiol. Pharmacol." Vol. 47, page 113, (1969)].

While, human GIP was isolated and purified by Valverde, I. et al who also determined a structure of the GIP ["FEBS" Vol. 172. pages 142 - 148, (1984)]. According to the description given in the literature, the human GIP consists of 42 amino acids and has molecular weight of about 5000. The GIP was considered to be a member of secretin family, since an amino acid sequence thereof is analogous with the secretin, VIP, glucagon and the like.

Each of said GIP is one extracted from animal organ and shows physiological actions which are generally classified into accelerating actions of gastric juice secretion and of insulin secretion. It has been known among them that the latter action remarkably increases, as the blood sugar concentration rises up. In view of this action, the GIP has been expected as it may become one of effective ingredients for curing a certain diabetes. Namely, among the diabetics, it is preferable for the insulin-independent patient, whose B cell function remains to lie in a physical state that insulin secretion is particularly accelerated, when his blood sugar concentration is higher.

The GIP according to the prior arts has been obtained through extraction from animal organ of pork, beef or man and thus it was quite difficult to obtain the same in a large amount. Namely, any of GIP has not actually been applied for clinicaly use, due to its poor productivity, in spite of that an effectiveness of GIP as the agent for curing diabetes has been expected.

A basic matter of the invention is in developing an industrially acceptable process for the preparation of human GIP and its related substances, by utilizing a so-called --Bio-Technologies--.

A principal object of the invention is to provide a cloned DNA comprising human GIP precursor or a fragment thereof.

An additional object of the invention is to provide a process for the preparation of the cloned DNA or the fragment thereof.

Another additional object of the invention is to provide a plasmid, wherein the cloned DNA or its fragment, for instance, human GIP per se or another physiologically active portion of the DNA is integrated, so that a microorganism transformed by the plasmid expresses the GIP or other physiologically active substances to allow a large scale production thereof.

The inventors carefully studied to finally obtain the cloned DNA for human GIP precursor and determine its structure, whereby the basic matter or problem has been dissolved. According to the invention, the principal object is attained by a cloned single-stranded DNA as defined in Claim 1 comprising 459 nucleotides which encodes an amino acid sequence for human GIP precursor, or a cloned DNA consisting of said single-strand DNA and its complementary single-strand DNA.

The DNA according to the invention has the following nucleotide sequence or any other nucleotide sequence same with the former in biological view point.

```
5'--- ATG GTG GCC ACG AAG ACC TTT GCT CTG CTG CTG CTG TCC CTG TTC

      CTG GCA GTG GGA CTA GGA GAG AAG AAA GAG GGT CAC TTC AGC GCT

      CTC CCC TCC CTG CCT GTT GGA TCT CAT GCT AAG GTG AGC AGC CCT

      CAA CCT CGA GGC CCC AGG TAC GCG GAA GGG ACT TTC ATC AGT GAC

      TAC AGT ATT GCC ATG GAC AAG ATT CAC CAA CAA GAC TTT GTG AAC

      TGG CTG CTG GCC CAA AAG GGG AAG AAG AAT GAC TGG AAA CAC AAC

      ATC ACC CAG AGG GAG GCT CGG GCG CTG GAG CTG GCC AGT CAA GCT
```

```
AAT AGG AAG GAG GAG GAG GCA GTG GAG CCA CAG AGC TCC CCA GCC

AAG AAC CCC AGC GAT GAA GAT TTG CTG CGG GAC TTG CTG ATT CAA

GAG CTG TTG GCC TGC TTG CTG GAT CAG ACA AAC CTC TGC AGG CTC

AGG TCT CGG ---3'
```

wherein A, C, G and T are, respectively, a deoxyribonucleotide having adenine, cytosine, guanine or thymine base and said sequence is given as that of each codon corresponding to a specified amino acid.

The term of --Nucleotide sequences same in biological view point--means a case of that even if, kinds or arrangement of nucleotides constituting codons, as --TTA-- and --CTG-- are different but each codon designates same amino acid (in this case, each of the codons designates --leucine--). In this case, therefore, the term means nucleotide sequences encoding following amino acid sequence which is designated by said nucleotide sequence formula.

```
Met-Val-Ala-Thr-Lys-Thr-Phe-Ala-Leu-Leu-Leu-Leu-Ser-Leu-Phe-

Leu-Ala-Val-Gly-Leu-Gly-Glu-Lys-Lys-Glu-Gly-His-Phe-Ser-Ala-

Leu-Pro-Ser-Leu-Pro-Val-Gly-Ser-His-Ala-Lys-Val-Ser-Ser-Pro-

Gln-Pro-Arg-Gly-Pro-Arg-Tyr-Ala-Glu-Gly-Thr-Phe-Ile-Ser-Asp-

Tyr-Ser-Ile-Ala-Met-Asp-Lys-Ile-His-Gln-Gln-Asp-Phe-Val-Asn-

Trp-Leu-Leu-Ala-Gln-Lys-Gly-Lys-Lys-Asn-Asp-Trp-Lys-His-Asn-

Ile-Thr-Gln-Arg-Glu-Ala-Arg-Ala-Leu-Glu-Leu-Ala-Ser-Gln-Ala-

Asn-Arg-Lys-Glu-Glu-Glu-Ala-Val-Glu-Pro-Gln-Ser-Ser-Pro-Ala-

Lys-Asn-Pro-Ser-Asp-Glu-Asp-Leu-Leu-Arg-Asp-Leu-Leu-Ile-Gln-

Glu-Leu-Leu-Ala-Cys-Leu-Leu-Asp-Gln-Thr-Asn-Leu-Cys-Arg-Leu-

Arg-Ser-Arg
```

According to the human GIP precursor cDNA having said nucleotide and amino acid sequences, it is found that the human GIP is encoded in a region of the 52nd to 93rd counting from the methionine (Met) residue designated by the starting codon of ATG and partitioned at both ends by the arginine (Arg) residue designated by the codon of AGG. The firstly arranged about 20 amino acids beginning from the initiative Met are considered as that corresponding to a signal peptide concerning to secretion, in view of its structure.

According to a process of the invention, the cloned double-strand DNA encoding human GIP precursor can be prepared by converting RNA extracted from upper small intestine (duodenum) into poly(A)RNA, constructing cDNA library with use of the poly(A)RNA and vector DNA to carry out a transformation of Escherichia coli (E. coli), while, previously synthesizing a mixture of 16 tetradecamers, each consisting of 14 deoxyribonucleotides of the formula

$$5'--- \ AT \begin{Bmatrix} C \\ T \end{Bmatrix} TT \begin{Bmatrix} A \\ G \end{Bmatrix} TCCAT \begin{Bmatrix} A \\ G \\ C \\ T \end{Bmatrix} GC \qquad (A)$$

which is complementary to mRNA corresponding to

Ala-Met-Asp-Lys-Ile

of 13th to 17th amino acids sequence from N-terminal in known amino acid sequence of human GIP and a mixture of 16 heptadecamers, each consisting of 17 deoxyribonucleotides of the formula

$$5' \text{---} \text{AT} \begin{bmatrix} A \\ G \end{bmatrix} \text{TT} \begin{bmatrix} A \\ G \end{bmatrix} \text{TG} \begin{bmatrix} C \\ T \end{bmatrix} \text{TTCCA} \begin{bmatrix} A \\ G \end{bmatrix} \text{TC} \qquad (B)$$

which is complementary to mRNA corresponding to

Asp-Trp-Lys-His-Asn-Ile

of 35th to 40th amino acids sequence from N-terminal in the known amino acid sequence for human GIP, labelling each synthesized oligodeoxyribonucleotide shown by said formulae (A) and (B) at 5'-terminal, screening said transformed E. coli by a hybridization using said labeled oligodeoxyribonucleotides as probes to obtain a positive clone which hybridizes both of the probes.

The ground that the 13th to 17th amino acids from N-terminal in amino acids for GIP is selected for the oligodeoxyribonucleotide (A) as one of probes and that the 35th to 40th amino acids from N-terminal in amino acids for GIP is selected for the oligodeoxyribonucleotide (B) as the other probe lies in making possible to respond for 5 or 6 kinds amino acids and minimum kinds of mRNA corresponding to such amino acids.

In the screening of transformed cells (E. coli), there is no difference in result, even if the hybridization with the oligodeoxyribonucleotides (A) is carried out prior to that with the other oligodeoxyribonucleotides (B), or vise versa.

A cloned single-strand DNA encoding human GIP region can be prepared by decomposing the resulting cloned double-strand DNA with use of a method known per se, for instance treating at 90°C for about 3 minutes and the cooling same with an ice bath.

The cloned single or double-strand DNA encoding human GIP precursor can be made into fragment in various length, by treating with a suitable restriction enzyme(s), binding fragments, synthesizing a region not obtaining a cleaving technique and binding and synthesized region to a fragment to make the desired fragment of human GIP region only or another physiologically active region.

The cloned double-strand DNA encoding human GIP precursor region or any fragment thereof may be integrated into a plasmid with a technique known per se, for instance taking out a plasmid from E. coli, purifying the plasmid, treating the plasmid with a restriction enzyme to cut the plasmid at a specified base position, and ligating with a DNA ligase the cloned DNA to the cleavage of the cut plasmid to re-construct a plasmid with the recombinant DNA.

The single-strand DNA encoding human GIP precursor or its fragment may be used as a probe to utilize same for a diagnosis in gene level on the diabetes not depending to insulin. A fragment of the DNA, which encodes an amino acid sequence other than GIP may be used to search a novel physiologically active peptide(s). The GIP precursor, GIP per se or other physiologically active substances can be produced in a large amount by transforming a microorganism or an eukaryotic cell with the plasmid integrated therein the double-strand DNA or a fragment thereof and culturing the microorganism or eukaryotic cell.

The invention will now be further explained with reference Examples and a Test Example for determining a structure of a cloned human GIP precursor, which will refer to drawings, wherein

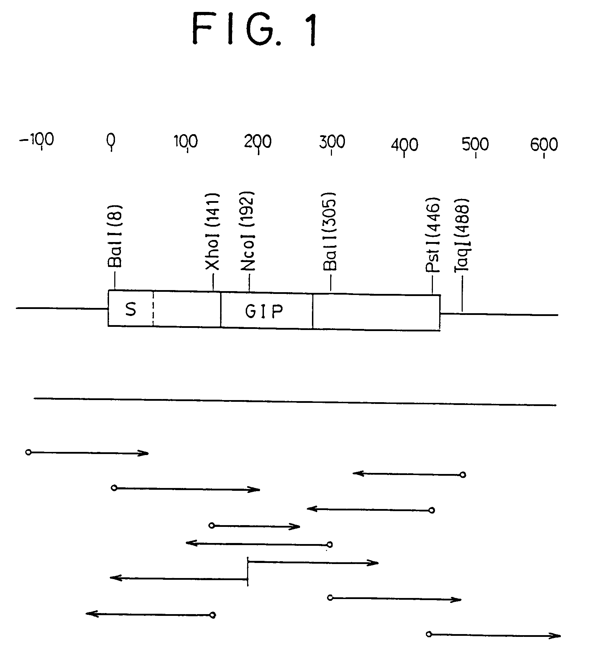

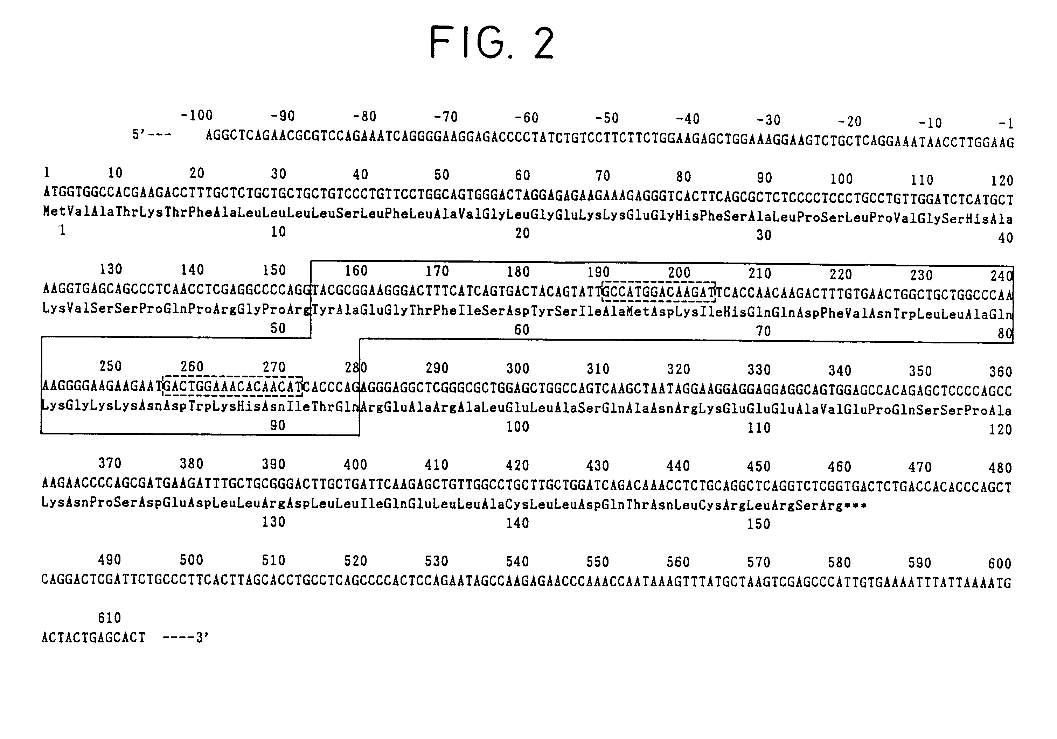

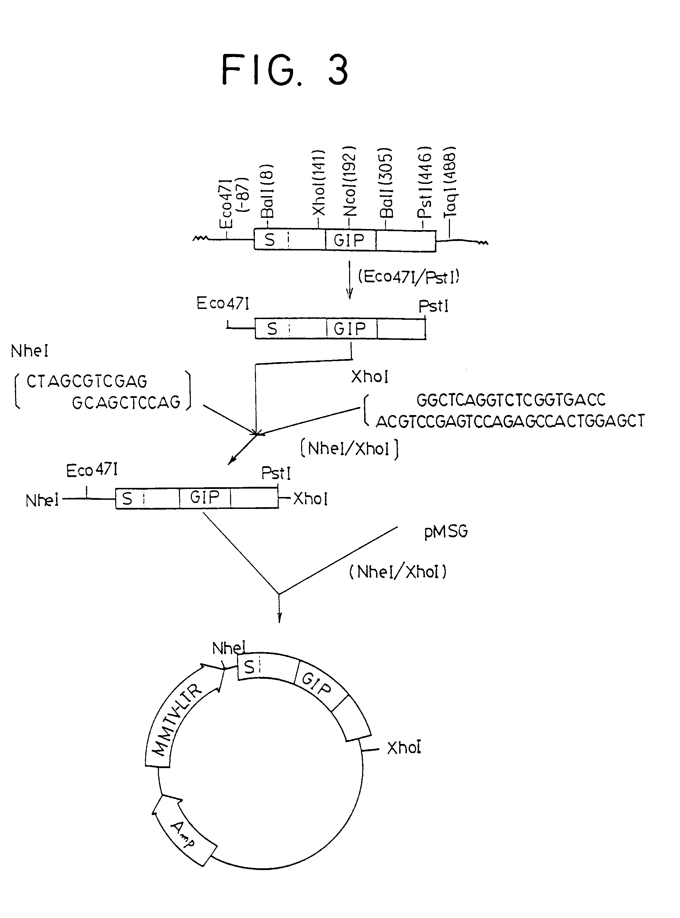

Fig. 1 is a strategy for sequencing cloned cDNA encoding the human GIP precursor according to the invention and a restriction enzyme map which displays only relevant restriction endonuclease sites; Fig. 2 shows a determined nucleotide sequence of cloned cDNA encoding human GIP precursor as well as an amino sequence corresponding to the nucleotide sequence in the human GIP precursor region; and Fig. 3 illustrates steps for preparing a plasmid, wherein the human GIP precursor is integrated.

Example 1

5

a) Preparation of poly(A)RNA comprising mRNA of human GIP

It has been known through a radio immunoassay and others that the human GIP presents at an upper small intestine and in other organs [ "American. J. Anatomy" Vol. 168, pages 109 - 118(1983)]. Therefore, a human duodinum obtained from a patient during pancreato-duodenectomy for pancreatic cancer was treated, in accordance with the method as disclosed by Chirgwin, J. M. et al [ "Biochemistry" Vol. 18, pages 5294 - 5299 (1979)]. Namely, the total RNA (1.2mg) was extracted from the tissue by 4M-guanidium thiocyanate, bound to an oligo(dT)cellulose column with use of 10mM-Tris hydrochloride buffer containing 0.5M KCl, as a binding buffer, and eluted with use of a buffer free from KC1 to obtain poly(A)RNA (150µg).

It has been confirmed through an electrophoresis that this RNA is not degradated, in view of RNA bands at 18s and 28s.

b) Construction of cDNA library

The human duodenal cDNA library was constructed with use of 30µg of said poly(A)RNA and 4.3µg of vector/primer DNA and in accordace with the method disclosed by Okayama, H. and Berg, P. ["Mol. Cell. Biol." Vol. 2. pages 161 - 170 (1982)] and E. coli (HB101) was transformed in accordance with the method disclosed by Morrison, D. A. ["Methods in Enzymol." Vol. 68, pages 326 - 331 (1979)]. Through a screening with use of LB-agar plate containing 40 µg/ml of ampicillin, ampicillin resistant transformants were obtained by 10000 cells per 1µg of poly(A)RNA, namely about 300000 cells in total.

c) Synthesis of oligodeoxyribonucleotide complementary to mRNA encoding human GIP

A mixture of 16 tetradecamers, each consisting of 14 deoxyribonucleotides of the formula

$$5'\text{---}\ AT\begin{Bmatrix}C\\T\end{Bmatrix}TT\begin{Bmatrix}A\\G\end{Bmatrix}TCCAT\begin{Bmatrix}A\\G\\C\\T\end{Bmatrix}GC \qquad (A)$$

which is complementary to mRNA corresponding to

Ala-Met-Asp-Lys-Ile

of the 13th to 17th amino acid sequence from N-terminal in known amino acid sequence for human GIP and a mixture of 16 heptadecamers, each consisting of 17 deoxyribonucleotides of the formula

$$5'\text{---}\ AT\begin{Bmatrix}A\\G\end{Bmatrix}TT\begin{Bmatrix}A\\G\end{Bmatrix}TG\begin{Bmatrix}C\\T\end{Bmatrix}TTCCA\begin{Bmatrix}A\\G\end{Bmatrix}TC \qquad (B)$$

which is complementary to mRNA corresponding to

Asp-Trp-Lys-His-Asn-Ile

of the 35th to 40th amino acid sequence from N-terminal in the known amino acid sequence of human GIP were synthesized in accordance with a modification method [Ito, N. et al "Nucleic Acids Res. "Vol. 10, pages 1755 - 1769 (1982)] of the Triester method ["Tetrahedron Letters" Vol. 28, page 2449 - 2452 (1978)].

These deoxyribonucleotides include all of possible mRNA sequences corresponding to said amino acid sequennces, excepting the terminal nucleotide residue corresponding to the 17th and 40th amino acid of --Ile--.

(d) cDNA Cloning

The cloning was carried out in accordance with the method as disclosed by Hanahan, D. et al ["Gene" Vol. 10, pages 63 - 67 (1980)].

Namely, about 30000 cells among said about 30000 ampicillin resistant transformants as described in said Item (b) were replicated on nitrocellulose filter, cultured for 3 to 4 hours on a agar plate containing 50$\mu$g of ampicillin and transferred on LB-agar plate containing 500$\mu$g of chloramphenicol to further culture the same at 37°C for one overnight. A colony formed on the filter was subjected to a bacteriolysis, namely a double-strand DNA was made into a single-strand DNA, fixed on a plate, neutralized to make pH to 7.5, and dipped the filter for 5 minutes in Tris-hydrochloride buffer (pH 7.5) containing 1.5M-NaCl to remove bacterial fragments and the like other than DNA. Thereafter, the filter was air driied and baked for 2 hours at 80°C to obtain a testing filter for screening, which is nitrocellulose filter carrying DNA due to about 30000 transformants.

While, the screening of the transformants was carried out as follows, in accordance with the method as disclosed by Grunstein, M. et al ["Proc. Natl. Acad. Sci. U.S.A." Vol. 72, pages 3961 - 3965 (1975)].

Each of the oligodeoxyribonucleotides synthesized by the method as stated in said Item (c) and shown by said formulae A and B was end-labeled at 5′-terminal with [$\gamma$-$^{32}$P]ATP (Amersham, 5000 Ci/mmol) and T$_4$ polynucleotide kinase (Toyobo Co. Ltd., Osaka, Japan) to make the oligodeoxyribonucleotides into probes for screening. A relative activity of each probe was 1 to 2 x 10$^6$ cpm/pmol.

The transformants on the testing filter were screened by a hybridization at 40°C for 18 hours and with use of the labeled oligodeoxyribonucreotides of the group shown by the Formula B, as the probes and judged by the autoradiogram method to find that only 6 transformants among about 30000 transformants are hybridizing positive clones. These positive clones were then screened by another hybridization at 36°C for 18 hours and with use of the labeled oligodeoxyribonucreotides of the group shown by the Formula A, as the probes and judged in the manner as above to find that there is only one positive clone.

This positive clone, namely human GIP precursor containing clone was analized by an electrophoresis to find that it has cDNA insert region of about 800bp including poly(dA)(dT) tails and poly(dG)(dC) tracts. This cloned DNA is double-strand one and nucleotide sequence thereof was elucidated as stated in the Test Example as stated below. Therefore, the DNA can be made into a fragment(s) in various length, by treating same with a suitable restriction enzyme(s) and if necessary, binding a synthetic DNA to the fragment to obtain a desired DNA fragment consisting of the GIP precursor region, GIP region or other region showing a certain physiological activity.

The double-strand DNA and double-strand DNA fragment or piece can be made into corresponding sigle-strand one by treating same at 90°C for about 3 minutes then then ice cooling to cause a decomposition thereof.

Test Example

(Determination of nucleotide sequence for cloned human GIP precursor and corresponding amino acid sequence)

A nucleotide sequence of cDNA insert region for the cloned human GIP precursor was determined directly or by sub-cloning to a pUC19 plasmid, in accordance with the method disclosed by Maxam, A. M. et al ["Methods in Enzymol." Vol. 65, pages 499 - 560 (1980)] and the method disclosed by Hattori, M. et al ["Anal. Biochem" Vol. 152, pages 232 - 238 (1980)] who employs a modified plasmid.

In Fig. 1, there are given a cDNA region of the human GIP precursor containing clone, restriction enzymes selected for determinng the nucleotide sequence in the region and a strategy for sequencing the cloned cDNA. The numerals at uppermost portion in the Figure are nucleotide number given as from the human GIP precursor region boxed at intermediate portion. The --Ball-- and the like are names of restriction enzymes and a numeral given in parentheses shows a cleavage portion or starting position (nucleotide number) for determining the nucleotide sequence. Among the boxed region for human GIP precursor, a region (S) shows that to be putative signal peptides, and another region (GIP) shows that already known as corresponding to the human GIP. In the lower portion in Fig. 1,each of horizontal arrow shows a direction and limit of the nucleotide sequence to be determined, due to the respective restriction enzyme. Each of the determination results carried out according to the method disclosed by Maxam, A. M. et al is shown by an arrow with a short vertical line which shows an isotope labeled position at 5′-terminal. While, each determination result carried out by the dideoxy method disclosed by Hattori, M. et al is shown by adding a small circle at an end of the arrow which indicates a starting position for nucleotide sequencing.

In Fig. 2, there are shown thus determined nucleotide sequence of cloned cDNA encoding the human GIP precursor as well as the amino acid sequence corresponding to the nucleotide sequence in the human GIP precursor region. In Fig. 2, the numeral given at upper side is the number of nucleotides as in Fig. 1, and another numeral given at lower side is number of amino acid residues in the human GIP precursor region. The region boxed with a solid line shows the known human GIP region, and each of regions boxed with broken line shows the regions employed as the probe for screening the transformants in Example 1.

As seen in Fig. 2, from the intiation codon of ATG for N-terminal methionine, there is an open reading frame for 153 amino acid residues before the first termination codon of TGA and the nucleotide sequence encoding known human GIP presents therein, which lies in the region from tyrosin (Tyr) in the 52nd position of the amino acid number to glutamine (Glu) in the 93rd position thereof. The N-terminal region, namely the 1st Met to 20th Leu seems to be a signal sequence (S region in Fig. 1) which may generally be found on secretory proteins, in view of its structure consisting of relative hydrophobic amino acids. In view of that the area corresponding to said GIP region is partitioned by basic amino acid of arginine (Arg), it seems to be that the GIP will be secreted in the form of the precursor and then cleaved by a protease in blood or the like to form the mature GIP. There is a canonical polyadenylation signal, AATAAA (nucleotide Nos. 553 - 558), located 55bp before the poly(A)tract. Such a sequence is usually located much closer to the site of polyadenylation and it is possible to say that the sequence ATTAAA (nucleotide Nos. 592 - 597) is polyadenylation sequence used in human GIP mRNA.

Example 2

(Preparation of plasmid integrating the human GIP precursor)

This embodiment will be explained with reference to the drawings and more particularly Fig. 3. In the first place, the c DNA clone for human GIP precursor as shown in Figs. 1 and 2 was treated with restriction enzymes Eco47I and PstI to obtain a fragment of about 530bp. On the other hand, NheI and XhoI linkers were prepared with use of a DNA synthesizer marketed by Pharmasia. The linkers were joined to Eco47I and XhoI sites of the fragment, respectively.

While, a commercially available plasmid (--pMSG-- marketed by Pharmasia) was treated with the restriction enzymes NheI and XhoI. To the resulting ends, said fragment with linkers was joined to re-construct into a plasmid, in which the human GIP precursor is integrated.

As shown in the last portion in Fig. 3, the resulting plasmid has the DNA clone of the human GIP precursor at down-stream of a powerful promoter of a long terminal repeat on mouse mammary tumor virus.

If L or CHO cells are transformed with use of such a plasmid and cultured, therefore, the human GIP precursor can easily be prepared in a large amount.

**Claims**

1. A cloned single-stranded DNA encoding human gastric inhibitory polypeptide precursor which has a single open reading frame comprising 459 deoxyribonucleotides and consisting essentially of the sequence of

```
5'--- ATG GTG GCC ACG AAG ACC TTT GCT CTG CTG CTG CTG TCC CTG
      TTC CTG GCA GTG GGA CTA GGA GAG AAG AAA GAG GGT CAC TTC
      AGC GCT CTC CCC TCC CTG CCT GTT GGA TCT CAT GCT AAG GTG
      AGC AGC CCT CAA CCT CGA GGC CCC AGG TAC GCG GAA GGG ACT
      TTC ATC AGT GAC TAC AGT ATT GCC ATG GAC AAG ATT CAC CAA
      CAA GAC TTT GTG AAC TGG CTG CTG GCC CAA AAG GGG AAG AAG
      AAT GAC TGG AAA CAC AAC ATC ACC CAG AGG GAG GCT CGG GCG
      CTG GAG CTG GCC AGT CAA GCT AAT AGG AAG GAG GAG GAG GCA
      GTG GAG CCA CAG AGC TCC CCA GCC AAG AAC CCC AGC GAT GAA
      GAT TTG CTG CGG GAC TTG CTG ATT CAA GAG CTG TTG GCC TGC
      TTG CTG GAT CAG ACA AAC CTC TGC AGG CTC AGG TCT CGG --- 3'
```

8

wherein A, C, G and T are, respectively, the deoxyribonucleotide having an adenine, cytosine, guanine or tymine base and the sequence is given as that of each codon encoding a specified amino acid, or any other nucleotide sequence with degeneracy to said sequence, or a double-stranded DNA consisting of said single-stranded DNA and its complementary single-stranded DNA.

2. A cloned single-stranded DNA or double-stranded DNA as claimed in Claim 1, wherein said single-stranded DNA encodes 153 amino acid residues consisting essentially of

```
Met-Val-Ala-Thr-Lys-Thr-Phe-Ala-Leu-Leu-Leu-Leu-Ser-Leu-
Phe-Leu-Ala-Val-Gly-Leu-Gly-Glu-Lys-Lys-Glu-Gly-His-Phe-
Ser-Ala-Leu-Pro-Ser-Leu-Pro-Val-Gly-Ser-His-Ala-Lys-Val-
Ser-Ser-Pro-Gln-Pro-Arg-Gly-Pro-Arg-Tyr-Ala-Glu-Gly-Thr-
```

```
Phe-Ile-Ser-Asp-Tyr-Ser-Ile-Ala-Met-Asp-Lys-Ile-His-Gln-
Gln-Asp-Phe-Val-Asn-Trp-Leu-Leu-Ala-gln-Lys-Gly-Lys-Lys-
Asn-Asp-Trp-Lys-His-Asn-Ile-Thr-Gln-Arg-Glu-Ala-Arg-Ala-
Leu-Glu-Leu-Ala-Ser-Gln-Ala-Asn-Arg-Lys-Glu-Glu-Glu-Ala-
Val-Glu-Pro-Gln-Ser-Ser-Pro-Ala-Lys-Asn-Pro-Ser-Asp-Glu-
Asp-Leu-Leu-Arg-Asp-Leu-Leu-Ile-Gln-Glu-Leu-Leu-Ala-Cys-
Leu-Leu-Asp-Gln-Thr-Asn-Leu-Cys-Arg-Leu-Arg-Ser-Arg.
```

3. A plasmid containing a double-stranded DNA which comprises a cloned single-stranded DNA encoding human gastric inhibitory peptide precursor which has a single open reading frame comprising 459 deoxyribonucleotides and consisting essentially of the sequence of

```
5'--- ATG GTG GCC ACG AAG ACC TTT GCT CTG CTG CTG CTG TCC CTG
      TTC CTG GCA GTG GGA CTA GGA GAG AAG AAA GAG GGT CAC TTC
      AGC GCT CTC CCC TCC CTG CCT GTT GGA TCT CAT GCT AAG GTG
      AGC AGC CCT CAA CCT CGA GGC CCC AGG TAC GCG GAA GGG ACT
      TTC ATC AGT GAC TAC AGT ATT GCC ATG GAC AAG ATT CAC CAA
      CAA GAC TTT GTG AAC TGG CTG CTG GCC CAA AAG GGG AAG AAG
      AAT GAC TGG AAA CAC AAC ATC ACC CAG AGG GAG GCT CGG GCG
      CTG GAG CTG GCC AGT CAA GCT AAT AGG AAG GAG GAG GAG GCA
      GTG GAG CCA CAG AGC TCC CCA GCC AAG AAC CCC AGC GAT GAA
      GAT TTG CTG CGG GAC TTG CTG ATT CAA GAG CTG TTG GCC TGC
      TTG CTG GAT CAG ACA AAC CTC TGC AGG CTC AGG TCT CGG --- 3'
```

wherein A, C, G and T are, respectively, the deoxyribonucleotide having an adenine, cytosine, guanine or tymine base and the sequence is given as that of each codon encoding a specified amino acid, or any other nucleotide sequence with degeneracy to said sequence, and its complementary single-stranded DNA, or a fragment of said double-stranded DNA.

4. A process for the preparation of a cloned single-stranded DNA comprising 459 deoxyribonucleotides which encodes human gastric inhibitory peptide precursor and a double-stranded DNA consisting of said cloned single-stranded DNA and its complementary single-stranded DNA, which process comprises steps of converting RNA extracted from upper small intestine into poly(A)RNA, constructing a cDNA library with use of said poly(A)RNA and vector DNA to carry out a transformation of Eschrechia coli, while previously synthesizing a mixture of 16 tetradecamers, each consisting of 14 deoxyribonucleotides of the formula

$$5' --- AT \begin{Bmatrix} C \\ T \end{Bmatrix} TT \begin{Bmatrix} A \\ G \end{Bmatrix} TCCAT \begin{Bmatrix} A \\ G \\ C \\ T \end{Bmatrix} GC \qquad (A)$$

which is complementary to mRNA corresponding to

Ala-Met-Asp-Lys-Ile

of 13th to 17th amino acids from N-terminal in an amino acid sequence known as that for human gastric inhibitory peptide and a mixture of 16 heptadecamers, each consisting of 17 deoxyribonucleotides of the formula

$$5' --- AT \begin{Bmatrix} A \\ G \end{Bmatrix} TT \begin{Bmatrix} A \\ G \end{Bmatrix} TG \begin{Bmatrix} C \\ T \end{Bmatrix} TTCCA \begin{Bmatrix} A \\ G \end{Bmatrix} TC \qquad (B)$$

which is complementary to mRNA corresponding to

Asp-Trp-Lys-His-Asn-Ile

of 35th to 40th amino acids from N-terminal in the amino acid sequence for the human gastric inhibitory peptide, labeling each synthesized oligodeoxyribonucleotide shown by said formulae (A) and (B) at 5'-terminal, screening said transformed Escherichian coli through a hybridization using said labeled oligodeoxyribonucleotides as probes to obtain the double-stranded DNA as positive clone which hybridizes to both of the prove groups, and if necessary separating the double-stranded DNA into its structural single-stranded DNAs.

**Patentansprüche**

1. Geklonte, einzelsträngige DNA, die für eine Inhibitor-Polypeptid-Vorstufe des menschlichen Magens kodiert und ein einzelnes, offenes Leseraster mit 459 Desoxyribonukleotiden enthält, im wesentlichen bestehend aus folgender Sequenz:

EP 0 269 072 B1

```
5'--- ATG GTG GCC ACG AAG ACC TTT GCT CTG CTG CTG CTG TCC CTG
      TTC CTG GCA GTG GGA CTA GGA GAG AAG AAA GAG GGT CAC TTC
      AGC GCT CTC CCC TCC CTG CCT GTT GGA TCT CAT GCT AAG GTG
      AGC AGC CCT CAA CCT CGA GGC CCC AGG TAC GCG GAA GGG ACT
      TTC ATC AGT GAC TAC AGT ATT GCC ATG GAC AAG ATT CAC CAA
      CAA GAC TTT GTG AAC TGG CTG CTG GCC CAA AAG GGG AAG AAG
      AAT GAC TGG AAA CAC AAC ATC ACC CAG AGG GAG GCT CGG GCG
      CTG GAG CTG GCC AGT CAA GCT AAT AGG AAG GAG GAG GAG GCA
      GTG GAG CCA CAG AGC TCC CCA GCC AAG AAC CCC AGC GAT GAA
      GAT TTG CTG CGG GAC TTG CTG ATT CAA GAG CTG TTG GCC TGC
      TTG CTG GAT CAG ACA AAC CTC TGC AGG CTC AGG TCT CGG --- 3'
```

wobei A, C, G und T für die jeweiligen Desoxyribonukleotide stehen, die eine Adenin-Base, Cytosin-Base, Guanin-Base oder eine Thymin-Base besitzen, und wobei die Sequenz in Codons angegeben ist, welche je eine spezifische Aminosäure kodieren;

oder irgendeine andere Nukleotidsequenz, die zur besagten Sequenz degeneriert ist; oder eine doppelsträngige DNA, die aus der besagten einzelsträngigen DNA und ihrer komplementären einzelsträngigen DNA besteht.

2.   Eine geklonte, einzelsträngige DNA oder doppelsträngige DNA, gemäß Anspruch 1, wobei die besagte einzelsträngige DNA 153 Aminosäure-Reste kodiert, im wesentlichen bestehend wie folgt:

```
Met-Val-Ala-Thr-Lys-Thr-Phe-Ala-Leu-Leu-Leu-Leu-Ser-Leu-
Phe-Leu-Ala-Val-Gly-Leu-Gly-Glu-Lys-Lys-Glu-Gly-His-Phe-
Ser-Ala-Leu-Pro-Ser-Leu-Pro-Val-Gly-Ser-His-Ala-Lys-Val-
Ser-Ser-Pro-Gln-Pro-Arg-Gly-Pro-Arg-Tyr-Ala-Glu-Gly-Thr-

Phe-Ile-Ser-Asp-Tyr-Ser-Ile-Ala-Met-Asp-Lys-Ile-His-Gln-
Gln-Asp-Phe-Val-Asn-Trp-Leu-Leu-Ala-gln-Lys-Gly-Lys-Lys-
Asn-Asp-Trp-Lys-His-Asn-Ile-Thr-Gln-Arg-Glu-Ala-Arg-Ala-
Leu-Glu-Leu-Ala-Ser-Gln-Ala-Asn-Arg-Lys-Glu-Glu-Glu-Ala-
Val-Glu-Pro-Gln-Ser-Ser-Pro-Ala-Lys-Asn-Pro-Ser-Asp-Glu-
Asp-Leu-Leu-Arg-Asp-Leu-Leu-Ile-Gln-Glu-Leu-Leu-Ala-Cys-
Leu-Leu-Asp-Gln-Thr-Asn-Leu-Cys-Arg-Leu-Arg-Ser-Arg.
```

3.   Plasmid, das eine doppelsträngige DNA enthält, umfassend eine geklonte, einzelsträngige DNA, die für eine Inhibitor-Polypeptid-Vorstufe des menschlichen Magens kodiert und ein einzelnes, offenes Leseraster mit 459 Desoxyribonukleotiden enthält, im wesentlichen bestehend aus folgender Sequenz:

```
5'--- ATG GTG GCC ACG AAG ACC TTT GCT CTG CTG CTG CTG TCC CTG
      TTC CTG GCA GTG GGA CTA GGA GAG AAG AAA GAG GGT CAC TTC
      AGC GCT CTC CCC TCC CTG CCT GTT GGA TCT CAT GCT AAG GTG
      AGC AGC CCT CAA CCT CGA GGC CCC AGG TAC GCG GAA GGG ACT
      TTC ATC AGT GAC TAC AGT ATT GCC ATG GAC AAG ATT CAC CAA
      CAA GAC TTT GTG AAC TGG CTG CTG GCC CAA AAG GGG AAG AAG
      AAT GAC TGG AAA CAC AAC ATC ACC CAG AGG GAG GCT CGG GCG
      CTG GAG CTG GCC AGT CAA GCT AAT AGG AAG GAG GAG GAG GCA
      GTG GAG CCA CAG AGC TCC CCA GCC AAG AAC CCC AGC GAT GAA
      GAT TTG CTG CGG GAC TTG CTG ATT CAA GAG CTG TTG GCC TGC
      TTG CTG GAT CAG ACA AAC CTC TGC AGG CTC AGG TCT CGG --- 3'
```

wobei A, C, G und T für die jeweiligen Desoxyribonukleotide stehen, die eine Adenin-Base, Cytosin-Base, Guanin-Base oder eine Thymin-Base besitzen, und wobei die Sequenz in Codons angegeben ist, welche je eine spezifische Aminosäure kodieren; oder irgendeine andere Nukleotidsequenz, die zur besagten Sequenz degeneriert ist, und ihre komplementäre einzelsträngige DNA, oder ein Fragment der besagten doppelsträngigen DNA.

4. Verfahren zur Herstellung einer geklonten, einzelsträngige DNA, die 459 Desoxyribonukleotide enthält und eine Inhibitor-Polypeptid-Vorstufe des menschlichen Magens kodiert und einer doppelsträngige DNA, die aus der besagten geklonten, einzelsträngigen DNA und ihrer komplementären einzeisträngigen DNA besteht, wobei das Verfahren folgende Stufen umfaßt:
- Umwandlung von RNA, extrahiert aus dem oberen Dünndarm, in poly(A)-RNA;
- Erstellung einer cDNA-Bibliothek unter Verwendung der besagten poly(A)-RNA und Vektor-DNA zur Durchführung einer Transformation von Escherichia coli, bei vorausgehender Synthese einer Mischung von 16 Tetradecameren, jedes bestehend aus 14 Desoxyribonukleotiden der Formel

$$5'--- AT\begin{Bmatrix}C\\T\end{Bmatrix} TT\begin{Bmatrix}A\\G\end{Bmatrix} TCCAT\begin{Bmatrix}A\\G\\C\\T\end{Bmatrix} GC \qquad (A)$$

welche komplementär zur mRNA ist, entsprechend

$$Ala-Met-Asp-Lys-Ile$$

der 13, bis 17. Aminosäure vom N-terminalen Ende einer Aminosäuresequenz die als jene des Inhibitor-Peptids des Magens bekannt ist, und einer Mischung von 16 Heptadecameren, jedes bestehend aus 17 Desoxyribonukleotiden der Formel

$$5'--- AT\begin{Bmatrix}A\\G\end{Bmatrix} TT\begin{Bmatrix}A\\G\end{Bmatrix} TG\begin{Bmatrix}C\\T\end{Bmatrix} TTCCA\begin{Bmatrix}A\\G\end{Bmatrix} TC \qquad (B)$$

welche komplementär zur mRNA ist, entsprechend

```
Asp-Trp-Lys-His-Asn-Ile
```

der 35, bis 40. Aminosäure vom N-terminalen Ende der Aminosäuresequenz des Inhibitor-Peptids des Magens;
- Markierung jedes synthetisierten Oligodesoxyribonukleotids der besagten Formeln (A) und (B) am 5'-terminalen Ende;
- Screening der besagten transformierten Escherichia coli durch eine Hybridisierung unter Verwendung der besagten markierten Oligodesoxyribonukleotiden als Sonden, um die doppelsträngige DNA als positiven Klon zu erhalten, welcher mit beiden Sonden-Gruppen hybridisiert; und
- falls erforderlich, Trennung der doppelsträngigen DNA in ihre einzelsträngigen DNA-Strukturen.

**Revendications**

1. ADN monocaténaire cloné, codant pour le précurseur du polypeptide inhibiteur gastrique humain, qui présente un unique cadre de lecture ouvert comprenant 459 désoxyribonucléotides et consistant essentiellement en la séquence :

```
5'--- ATG GTG GCC ACG AAG ACC TTT GCT CTG CTG CTG CTG TCC CTG
      TTC CTG GCA GTG GGA CTA GGA GAG AAG AAA GAG GGT CAC TTC
      AGC GCT CTC CCC TCC CTG CCT GTT GGA TCT CAT GCT AAG GTG
      AGC AGC CCT CAA CCT CGA GGC CCC AGG TAC GCG GAA GGG ACT
      TTC ATC AGT GAC TAC AGT ATT GCC ATG GAC AAG ATT CAC CAA
      CAA GAC TTT GTG AAC TGG CTG CTG GCC CAA AAG GGG AAG AAG
      AAT GAC TGG AAA CAC AAC ATC ACC CAG AGG GAG GCT CGG GCG
      CTG GAG CTC GCC AGT CAA GCT AAT AGG AAG GAG GAG GAG GCA
      GTG GAG CCA CAG AGC TCC CCA GCC AAG AAC CCC AGC GAT GAA
      GAT TTG CTG CGG GAC TTG CTG ATT CAA GAG CTG TTG GCC TGC
      TTG CTG GAT CAG ACA AAC CTC TGC AGG CTC AGG TCT CGG --- 3'
```

dans laquelle A, C, G et T représentent respectivement les désoxyribonucléotides comptant la base respectivement adénine, cytosine, guanine ou thymine, et la séquence est donnée comme celle de chaque codon codant pour un acide aminé spécifié,
ou en tout autre séquence nucléotidique ayant une dégénérescence par rapport à ladite séquence,
ou ADN bicaténaire consistant en ledit ADN monocaténaire et son ADN monocaténaire complémentaire.

2. ADN monocaténaire cloné ou ADN bicaténaire selon la revendication 1, dans lequel ledit ADN monocaténaire code pour 153 résidus acides aminés consistant essentiellement en :

```
Met-Val-Ala-Thr-Lys-Thr-Phe-Ala-Leu-Leu-Leu-Leu-Ser-Leu-
Phe-Leu-Ala-Val-Gly-Leu-Gly-Glu-Lys-Lys-Glu-Gly-His-Phe-
Ser-Ala-Leu-Pro-Ser-Leu-Pro-Val-Gly-Ser-His-Ala-Lys-Val-
Ser-Ser-Pro-Gln-Pro-Arg-Gly-Pro-Arg-Tyr-Ala-Glu-Gly-Thr-
```

13

```
Phe-Ile-Ser-Asp-Tyr-Ser-Ile-Ala-Met-Asp-Lys-Ile-His-Gln-
Gln-Asp-Phe-Val-Asn-Trp-Leu-Leu-Ala-gln-Lys-Gly-Lys-Lys-
Asn-Asp-Trp-Lys-His-Asn-Ile-Thr-Gln-Arg-Glu-Ala-Arg-Ala-
Leu-Glu-Leu-Ala-Ser-Gln-Ala-Asn-Arg-Lys-Glu-Glu-Glu-Ala-
Val-Glu-Pro-Gln-Ser-Ser-Pro-Ala-Lys-Asn-Pro-Ser-Asp-Glu-
Asp-Leu-Leu-Arg-Asp-Leu-Leu-Ile-Gln-Glu-Leu-Leu-Ala-Cys-
Leu-Leu-Asp-Gln-Thr-Asn-Leu-Cys-Arg-Leu-Arg-Ser-Arg.
```

3. Plasmide contenant un ADN bicaténaire qui comprend un ADN monocaténaire cloné, codant pour le précurseur du peptide inhibiteur gastrique humain, qui présente un unique cadre de lecture ouvert comprenant 459 désoxyribonucléotides et consistant essentiellement en la séquence :

```
5'--- ATG GTG GCC ACG AAG ACC TTT GCT CTG CTG CTG CTG TCC CTG
      TTC CTG GCA GTG GGA CTA GGA GAG AAG AAA GAG GGT CAC TTC
      AGC GCT CTC CCC TCC CTG CCT GTT GGA TCT CAT GCT AAG GTG
      AGC AGC CCT CAA CCT CGA GGC CCC AGG TAC GCG GAA GGG ACT
      TTC ATC AGT GAC TAC AGT ATT GCC ATG GAC AAG ATT CAC CAA
      CAA GAC TTT GTG AAC TGG CTG CTG GCC CAA AAG GGG AAG AAG
      AAT GAC TGG AAA CAC AAC ATC ACC CAG AGG GAG GCT CGG GCG
      CTG GAG CTG GCC AGT CAA GCT AAT AGG AAG GAG GAG GAG GCA
      GTG GAG CCA CAG AGC TCC CCA GCC AAG AAC CCC AGC GAT GAA
      GAT TTG CTG CGG GAC TTG CTG ATT CAA GAG CTG TTG GCC TGC
      TTG CTG GAT CAG ACA AAC CTC TGC AGG CTC AGG TCT CGG --- 3'
```

dans laquelle A, C, G et T représentent respectivement les désoxyribonucléotides comportant la base adénine, cytosine, guanine ou thymine, et la séquence est donnée comme celle de chaque codon codant pour un acide aminé spécifié,
ou en tout autre séquence nucléotidique ayant une dégénérescence par rapport à ladite séquence, et son ADN monocaténaire complémentaire, ou un fragment dudit ADN bicaténaire.

4. Procédé de préparation d'un ADN monocaténaire cloné, comprenant 459 désoxyribonucléotides, qui code pour le précurseur du peptide inhibiteur gastrique humain, et d'un ADN bicaténaire consistant en ledit ADN monocaténaire cloné et son ADN monocaténaire complémentaire, lequel procédé comprend les étapes consistant à :
   - convertir l'ARN extrait de l'intestin grêle supérieur en ARN poly(A) ;
   - construire une banque d'ADNc à l'aide dudit ARN poly(A) et d'un ADN vecteur, pour effectuer une transformation b'Escherichia coli, tout en synthétisant au préalable un mélange de 16 tétradécamères, consistant chacun en 14 désoxyribonucléotides de la formule :

$$5'--- AT \begin{Bmatrix} C \\ T \end{Bmatrix} TT \begin{Bmatrix} A \\ G \end{Bmatrix} TCCAT \begin{Bmatrix} A \\ G \\ C \\ T \end{Bmatrix} GC \qquad (A)$$

14

qui est complémentaire de l'ARNm correspondant à :

## Ala-Met-Asp-Lys-Ile

du 13$^{\text{ème}}$ au 17$^{\text{ème}}$ acides aminés à partir de l'extrémité N-terminale dans une séquence d'acides aminés connue comme étant celle du peptide inhibiteur gastrique humain, et un mélange de 16 heptadécamères, consistant chacun en 17 désoxyribonucléotides de la formule :

$$5' - - - \ AT \begin{Bmatrix} A \\ G \end{Bmatrix} \ TT \begin{Bmatrix} A \\ G \end{Bmatrix} \ TG \begin{Bmatrix} C \\ T \end{Bmatrix} \ TTCCA \begin{Bmatrix} A \\ G \end{Bmatrix} \ TC \qquad (B)$$

qui est complémentaire de l'ARNm correspondant à

## Asp-Trp-Lys-His-Asn-Ile

du 35$^{\text{ème}}$ au 40$^{\text{ème}}$ acides aminés à partir de l'extrémité N-terminale dans la séquence d'acides aminés pour le peptide inhibiteur gastrique humain ;

- marquer chaque oligodésoxyribonucléotide synthétisé, représenté par lesdites formules (A) et (B) à l'extrémité 5';
- sélectionner ledit Escherichia coli transformé par une hybridation à l'aide desdits oligodésoxyribonucléotides marqués en tant que sondes pour obtenir l'ADN bicaténaire en tant que clone positif qui s'hybride aux deux sondes ; et
- si nécessaire, séparer l'ADN bicaténaire en ses ADN monocaténaires structuraux.

# FIG. 1

# FIG. 2

EP 0 269 072 B1

```
          -100        -90        -80        -70        -60        -50        -40        -30        -20        -10        -1
   5'---      AGGCTCAGAACGCGTCCAGAAATCAGGGGAAGGAGACCCCTATCTGTCCTTCTTCTGGAAGAGCTGGAAAGGAAGTCTGCTCAGGAAATAACCTTGGAAG


1         10         20         30         40         50         60         70         80         90        100        110        120
ATGGTGGCCACGAAGACCTTTGCTCTGCTGCTGCTGTCCCTGTTCCTGGCAGTGGGACTAGGAGAGAAGAAAGAGGGTCACTTCAGCGCTCTCCCCTCCCTGCCTGTTGGATCTCATGCT
MetValAlaThrLysThrPheAlaLeuLeuLeuLeuSerLeuPheLeuAlaValGlyLeuGlyGluLysLysGluGlyHisPheSerAlaLeuProSerLeuProValGlySerHisAla
 1                            10                            20                            30                            40


         130        140        150        160        170        180        190        200        210        220        230        240
AAGGTGAGCAGCCCTCAACCTCGAGGCCCCAGGTACGCGGAAGGGACTTTCATCAGTGACTACAGTATTGCCATGGACAAGATTCACCAACAAGACTTTGTGAACTGGCTGCTGGCCCAA
LysValSerSerProGlnProArgGlyProArgTyrAlaGluGlyThrPheIleSerAspTyrSerIleAlaMetAspLysIleHisGlnGlnAspPheValAsnTrpLeuLeuAlaGln
                            50                            60                            70                            80


         250        260        270        280        290        300        310        320        330        340        350        360
AAGGGGAAGAAGAATGACTGGAAACACAACATCACCCAGAGGGAGGCTCGGGCGCTGGAGCTGGCCAGTCAAGCTAATAGGAAGGAGGAGGAGGCAGTGGAGCCACAGAGCTCCCCAGCC
LysGlyLysLysAsnAspTrpLysHisAsnIleThrGlnArgGluAlaArgAlaLeuGluLeuAlaSerGlnAlaAsnArgLysGluGluGluAlaValGluProGlnSerSerProAla
                            90                           100                           110                           120


         370        380        390        400        410        420        430        440        450        460        470        480
AAGAACCCCAGCGATGAAGATTTGCTGCGGGACTTGCTGATTCAAGAGCTGTTGGCCTGCTTGCTGGATCAGACAAACCTCTGCAGGCTCAGGTCTCGGTGACTCTGACCACACCCAGCT
LysAsnProSerAspGluAspLeuLeuArgAspLeuLeuIleGlnGluLeuLeuAlaCysLeuLeuAspGlnThrAsnLeuCysArgLeuArgSerArg***
                            130                           140                           150


         490        500        510        520        530        540        550        560        570        580        590        600
CAGGACTCGATTCTGCCCTTCACTTAGCACCTGCCTCAGCCCCACTCCAGAATAGCCAAGAGAACCCAAACCAATAAAGTTTATGCTAAGTCGAGCCCATTGTGAAAATTTATTAAAATG


         610
ACTACTGAGCACT    ----3'
```

# FIG. 3